# DEMANDE DE BREVET EUROPEEN

(11) **EP 2 441 435 A1**
(43) Date de publication de la demande: **18.04.2012**
(21) Numéro de dépôt: 11185485.7
(22) Date de dépôt: 17.10.2011
(51) Int. Cl.: A61K 8/97, A61K 36/36, A61Q 19/00

(54) **Utilisation d'une composition cosmétique comprenant un extrait de dianthus carthusianorum**

(30) Priorité: 18.10.2010 FR 1004090
(71) Demandeur: Laboratoires Clarins, 92200 Neuilly sur Seine (FR)
(72) Inventeur: Courtin, Olivier, 92200 NEUILLY SUR SEINE (FR)
(74) Mandataire: Mena, Sandra

(57) **Abrégé**

La présente invention concerne l'utilisation d'une composition cosmétique comprenant un extrait de *Dianthus carthusianorum* pour le soin des peaux grasses. La présente invention concerne aussi un procédé de traitement cosmétique pour prévenir et/ou traiter les problèmes des peaux grasses, comprenant l'application sur la peau d'une composition cosmétique comportant un extrait de *Dianthus carthusianorum.* La présente invention concerne enfin un extrait de *Dianthus carthusianorum* pour son utilisation comme agent régulateur de la production de sébum et/ou comme agent régulateur de la pour la prévention et/ou le traitement des peaux grasses.

## Description

La présente invention concerne l'utilisation d'une composition cosmétique pour prévenir et/ou traiter les problèmes des peaux grasses.

On peut distinguer selon l'hydratation de la couche cornée et sa production de sébum, trois principaux types de peau : les peaux sèches, les peaux grasses et les peaux mixtes. La connaissance des différents types de peau permet d'adapter un traitement cosmétique approprié pour chaque cas.

La peau est constituée, entre autre, de glandes sébacées situées au niveau du derme et presque toujours associées à un poil. Toutes les parties du corps en sont pourvues, à l'exception de la plante des pieds et de la paume des mains. Certaines zones comme le visage, le cuir chevelu, le torse en comportent un plus grand nombre (environ cinq millions). Ces glandes sébacées sécrètent le sébum qui s'écoule le long du canal pilaire jusqu'à la surface de la peau où, au contact de la sueur, il forme le film hydrolipidique qui hydrate la couche cornée et la protège.

La glande sébacée est une glande acineuse en grappe. Elle est constituée de nombreuses couches cellulaires dans lesquelles on trouve deux types de cellules. D'une part des cellules indifférenciées (couche germinative), situées vers la périphérie de la glande, qui se divisent activement. Ces cellules migrent en 2 semaines environ vers le centre pour donner des cellules différenciées. D'autre part des cellules différenciées centrales (sébocytes) qui contiennent l'équipement enzymatique nécessaire à la synthèse des lipides. Ces cellules ne se divisent plus. En 8 jours elles se transforment en cellules matures plus grosses, remplies de sébum ; les lipides y sont synthétisés et stockés pour constituer finalement de grosses vacuoles.

Dans l'espèce humaine, chaque follicule se développe selon son propre cycle, c'est à dire qu'une glande peut s'atrophier pendant qu'une autre s'hypertrophie. Le renouvellement des sébocytes est d'environ 3 semaines.

Le volume glandulaire dépend de l'activité proliférative du compartiment germinatif, du temps nécessaire à la différenciation du sébocyte et de la qualité du sébum synthétisé par chaque sébocyte.

Le sébum est produit en quantité plus ou moins importante, en fonction de la taille et du nombre des glandes sébacées. Une sécrétion trop abondante entraîne une modification de la peau : elle devient grasse avec un aspect luisant, son grain est épais et ses pores dilatés avec parfois des points noirs. La glande sébacée, annexe du follicule pileux, est le siège d'une intense activité cellulaire et métabolique et en particulier de la synthèse de sébum. Sa synthèse est l'objet d'une régulation et d'une sensibilité hormonale très fine. Le sébum est constitué au niveau de l'infundibulum pilaire de cires (26%), de triglycérides (60%), de squalènes (12%) et de cholestérol (3%).

La quantité de lipides du sébum au niveau de la peau grasse est de 500 à 800 µg/cm² contrairement à la peau normale où elle se situe entre 200 et 400 µg/cm². A ce sébum s'ajoutent les lipides d'origine épidermique type stérols, céramides et esters de stérols.

Les variations moindres de la séborrhée concernent le concept cosmétologique de la peau grasse en opposition à la peau sèche. Les variations plus importantes de la séborrhée peuvent jouer un rôle notable dans l'acné, la dermatite séborrhéique et l'alopécie androgénique.

Le contrôle hormonal de la régulation et de la prolifération des sébocytes et de la sécrétion sébacée est évident. Les androgènes représentent le principal stimulus. Chez l'homme, c'est la testostérone d'origine testiculaire alors que chez la femme ce sont la DHEA (dihydroépiandrostérone) d'origine surrénalienne et la delta 4 androstènedione d'origine ovarienne. La glande sébacée possède l'équipement enzymatique pour former la dihydrotestostérone (DHT) par l'intermédiaire d'une enzyme la 5α-réductase. La DHT stimule l'activité de la glande sébacée en favorisant la prolifération des sébocytes.

La ligne médiane du visage est plus riche en glandes sébacées que le reste du visage. Le front, le nez mais aussi le cuir chevelu et la partie médiane du dos et du torse sont également très riches en glandes sébacées. Le sébum, produit en excès, entraine à terme un épaississement de la couche cornée. Les pores s'obstruent, entrent en contact avec les bactéries, à l'origine des comédons plus ou moins inflammés.

Outre le caractère peu esthétique d'une peau brillante, il convient de remarquer que les peaux grasses présentent aussi une tendance à être facilement irritables et une très mauvaise tenue du maquillage, elles sont plus fragiles et ont tendance à se déshydrater. Il existe donc un besoin pour une composition cosmétique capable de limiter la production de sébum afin de conserver à la surface cutanée ses caractéristiques de « peau normale ». On a proposé dans l'art antérieur plusieurs compositions cosmétiques susceptibles d'offrir une solution aux problèmes des peaux grasses (voir le brevet FR 2 795 321). Ces compositions contiennent souvent des poudres qui permettent d'absorber le sébum et donc de matifier la peau par un effet mécanique, ou des agents kératolitiques favorisant l'expulsion des bouchons cornés. Il existe aussi des actifs astringents permettant de lutter contre la dilatation des follicules sébacés, ou des actifs exfoliants permettant de diminuer l'épaisseur de la peau.

Toutefois, l'efficacité de ces compositions est relative, car la plupart ne limitent pas la production de sébum mais ne font qu'absorber le sébum par un effet de pompage. En outre, la plupart de ces compositions n'ont qu'un effet curatif et ne permettent pas de prévenir à plus long terme le phénomène de surproduction de sécrétion sébacée. D'autre part, le soufre actif efficace sur la régularisation de la production de sébum n'est généralement pas utilisable dans une composition cosmétique du fait de sa mauvaise odeur.

Il subsiste donc le besoin d'une composition cosmétique qui permet de prévenir et traiter les problèmes des peaux grasses, une telle composition se devant d'agir sur les causes des peaux grasses et non sur leurs symptômes.

La demanderesse a mis en évidence l'activité inhibitrice d'un extrait de *Dianthus carthusianorum* sur la 5α-réductase et donc sur la production de sébum. En effet, un extrait de *Dianthus carthusianorum* étant capable d'inhiber la 5α-réductase, la glande sébacée produit moins de dihydro-testostérone (DHT). Par voie de conséquence la production de sébocytes étant amoindrie, la quantité de sébum déversée par les glandes sébacées à la surface cutanée est diminuée. La peau ne présente alors plus les caractéristiques inesthétiques d'une peau grasse. Un extrait de *Dianthus carthusianorum* possède une action à la fois curative et préventive puisqu'il permet de moduler à long terme la production de sébum.

La présente invention concerne donc l'utilisation d'une composition cosmétique comprenant un extrait de *Dianthus carthusianorum* pour le soin des peaux grasses.

La présente invention concerne aussi l'utilisation d'une composition cosmétique comprenant un extrait de *Dianthus carthusianorum* comme agent régulateur des peaux grasses.

La présente invention concerne également l'utilisation d'une composition cosmétique comprenant un extrait de *Dianthus carthusianorum* comme agent régulateur de la production de sébum.

La présente invention concerne également l'utilisation d'une composition cosmétique comprenant un extrait de *Dianthus carthusianorum* comme agent régulateur de la prolifération des sébocytes.

La présente invention concerne également l'utilisation d'une composition cosmétique comprenant un extrait de *Dianthus carthusianorum* comme agent purifiant et/ou comme agent matifiant.

La présente invention concerne enfin l'utilisation d'une composition cosmétique comprenant un extrait de *Dianthus carthusianorum* comme agent protecteur anti-brillance.

Le *Dianthus carthusianorum,* également connu sous le nom d'oeillet des chartreux, est une plante de la famille des caryophyllacées. C'est une plante vivace de 20 à 60 cm originaire d'Europe ou d'Asie tempérée. De la touffe aux feuilles herbacées gris-vert émergent des tiges dressées portant les inflorescences de 4 à 6 fleurs rose-pourpre qui s'épanouissent tout l'été. Le *Dianthus carthusianorum* est une plante riche en saponines et en flavonoïdes, majoritairement de la rutine, des dérivés de la lutéoline, de l'orientine, de la quercétine et du kaempférol pouvant également être présents.

De façon avantageuse, l'extrait de *Dianthus carthusianorum* utilisable dans le cadre de la présente invention est un extrait des parties aériennes de la plante (tiges, feuilles, fleurs). Il est possible de se fournir en *Dianthus carthusianorum* (ou oeillet des chartreux) auprès de Sociétés telles que Jardin Jasmin ou Biozart.

L'extrait de *Dianthus carthusianorum* est obtenu par la mise en oeuvre d'un procédé comprenant au moins une étape d'extraction de l'extrait à l'aide d'éthanol à 60% et au moins une étape de distillation de l'éthanol sous vide. Le concentré aqueux ainsi obtenu est formulé sur glycérine et filtré. Il s'agit d'un liquide limpide à légèrement opalescent de couleur ambrée à marron et d'odeur caractéristique présentant les caractéristiques suivantes :
- pH = 3,5 à 5
- densité relative = 1,100 à 1,250
- index de réfraction = 1,400 à 1,420
- flavonoïdes ≥ 0,05%
- rapport plante/extrait : 1/10

De préférence la composition cosmétique ou dermatologique selon l'invention comprend de 0,0001 à 1% d'extrait de *Dianthus carthusianorum,* et plus préférentiellement de 0,01 à 1%, en poids de matière sèche par rapport au poids total de la composition.

Selon l'invention, l'extrait de *Dianthus carthusianorum* peut être utilisé avec un ou plusieurs autres actifs ayant des propriétés anti-peaux grasses. En particulier, l'extrait de *Dianthus carthusianorum* peut être associé dans une composition cosmétique ou dermatologique à de la chrysine, aussi dénommée 5,7-dihydroxyflavone, molécule capable de réguler la prolifération des sébocytes humains, et donc la production de sébum.

De même, l'extrait de *Dianthus carthusianorum* peut être associée dans une composition cosmétique ou dermatologique à un extrait de Lamier blanc, aussi appelé *Lamium album.* En effet, le lamier blanc présente une teneur en soufre supérieure à 200 ppm. Comme le soufre est capable de réguler la production de sébum, l'utilisation d'un extrait de Lamier blanc, et plus particulièrement d'un extrait glycolique, permet de disposer de soufre dans la composition cosmétique de l'invention sans présenter d'inconvénient au niveau de l'odorat. Il peut aussi s'agir d'une fraction d'un extrait de Lamier blanc riche en soufre dès lors, bien entendu, que cette fraction ne présente pas d'odeur désagréable.

Enfin, l'extrait de *Dianthus carthusianorum* peut être associée dans une composition cosmétique ou dermatologique à un extrait *d'hamamélis virginiana* connu pour ses propriétés astringentes, et/ou à du chlorhydrate de pyridoxine ou vitamine B6, qui réduit le flux de sébum et normalise le pH des peaux grasses, et/ou à des dérivés de zinc qui possèdent à la fois une activité astringente et une efficacité fongicide et bactéricide reconnue. On entend par dérivés de zinc les sels de zinc, et en particulier le sulfate de zinc et le gluconate de zinc.

La composition cosmétique de la présente invention à application topique peut constituer notamment une composition de protection, de nettoyage, de traitement ou de soin cosmétique ou dermatologique pour le visage, pour le cou, ou pour le corps, comme par exemple : crèmes de jour, crèmes de nuit, lotions pour le visage, masques, gels nettoyants moussants, laits corporels, une composition capillaire (par exemple une lotion pour le cuir chevelu), ou une composition de maquillage (par exemple fond de teint, crème teintée).

La composition cosmétique selon la présente invention peut contenir un ou plusieurs autres composants connus de l'homme du métier, tels que des agents de formulation ou additifs d'usage connu et classique dans les compositions cosmétiques.

A titre d'exemple et de façon non limitative, de tels agents de formulation et additifs peuvent être des gélifiants hydrophiles ou lipophiles, des adoucissants, des colorants, des agents solubilisants, des agents de texture, des parfums, des charges, des actifs filmogènes, des conservateurs, des tensioactifs, des émulsionnants, des huiles, des glycols, des vitamines, des filtres solaires, etc. Grâce à ses connaissances en matière de cosmétiques, l'homme du métier saura quels agents de formulation ajouter à la composition cosmétique selon l'invention et en quelles quantités, en fonction des propriétés recherchées.

De plus, la composition cosmétique selon la présente invention peut se présenter sous toute forme connue de l'homme du métier dans le domaine de la cosmétique sans aucune autre restriction galénique particulière que celle pour l'application sur la peau. Ainsi, la composition cosmétique selon l'invention peut avoir la forme d'une solution ou suspension aqueuse, alcoolique ou d'une suspension huileuse ou d'une solution ou d'une dispersion de type lotion ou sérum, d'une émulsion de consistance liquide ou semi-liquide de type lait, obtenues par dispersion d'une phase grasse dans une phase aqueuse (émulsion Huile dans Eau : H/E) ou inversement (Eau dans Huile : E/H), ou d'un gel, d'une lotion, d'un masque. On peut également envisager les formulations cosmétiques selon l'invention sous la forme d'une mousse ou encore sous forme de compositions pour aérosol comprenant également un agent propulseur sous pression.

La présente invention concerne également un procédé de traitement cosmétique pour prévenir et/ou traiter les peaux grasses, comprenant l'application sur la peau d'une composition cosmétique comportant un extrait de *Dianthus carthusianorum.*

La présente invention concerne aussi un extrait de *Dianthus carthusianorum* pour son utilisation comme agent régulateur de la production de sébum et/ou comme agent régulateur de la prolifération des sébocytes pour la prévention et/ou le traitement des peaux grasses.

Les exemples ci-après concernent, d'une part l'évaluation de l'effet d'un l'extrait de *Dianthus carthusianorum* sur l'activité de la 5α-réductase, ainsi que des compositions objets de la présente invention.

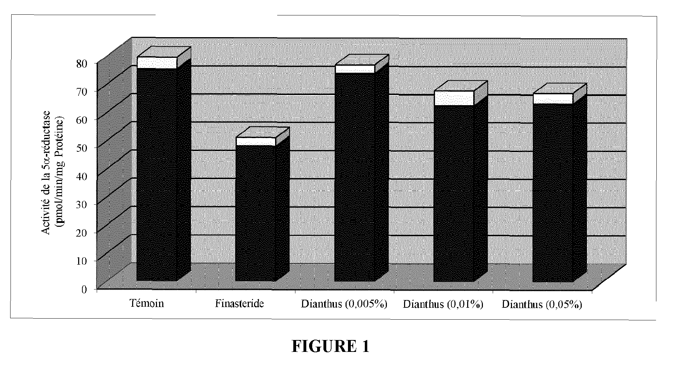

Les exemples font également références à la Figure 1 qui représente l'activité de la 5α-réductase exprimée en pmol/min/mg protéine en présence de finastéride - témoin positif - et en présence d'extrait de *Dianthus carthusianorum* à différentes concentrations.

### I. EVALUATION DE L'INHIBITION DE LA 5α-REDUCTASE IN SITU DANS L'EPIDERME RECONSTITUE SKINETHIC^{®}.

### A. MATERIEL ET METHODE

### 1. Traitement des épidermes

On travaille sur des épidermes reconstitués SKINETHIC^{®}. Des kératinocytes d'origine humaine sont ensemencés sur des filtres en polycarbonate de 0,5 cm² dans un milieu défini MCDB153 modifié : Molecular, Cellular and Developmental Biology (Biologie du dévelopement moléculaire et cellulaire 153 modifié) et supplémenté. Les cellules sont cultivées pendant 14 jours à l'interface air/liquide, le milieu de culture étant changé tous les deux jours. Les épidermes ainsi formés sont utilisés pour la réalisation de l'étude à partir du 17^{ème} jour de la culture.

L'essai correspondant aux traitements des épidermes reconstitués, est conduit pendant 24 heures et est réalisé comme suit :
Lot 1 : 3 épidermes témoins non traités,
Lot 2 : 3 épidermes traités par le témoin positif (Finastéride 10⁻⁵ M),
Lot 3 : 3 épidermes traités par l'extrait de *Dianthus carthusianorum* à 0,005%,
Lot 4 : 3 épidermes traités par l'extrait de *Dianthus carthusianorum* à 0,01%,
Lot 5 : 3 épidermes traités par l'extrait de *Dianthus carthusianorum* à 0,05%.

Le produit à l'étude et le témoin positif sont appliqués à la surface de chaque épiderme traité à raison de 10 µl/cm² pendant 24 heures. La [³H]-testostérone est ensuite appliquée sur les épidermes à raison de 5 µci/épiderme. Les épidermes sont incubés pendant 4 heures à 37°C dans un incubateur à CO₂ (5%).

### 2. Méthode de dosage

A la fin du temps d'incubation (4 heures), les milieux de culture sont récupérés et soumis directement à l'extraction organique dans l'éther diéthylique. 3 mL d'éther diéthylique sont ajoutés à chaque échantillon pour l'extraction des métabolites de la testostérone. Cette étape est répétée 3 fois pour optimiser l'extraction. Les différentes phases organiques sont rassemblées puis évaporées à sec. Les différents extraits sont ensuite reconstitués dans 10 µL d'éthanol pour la réalisation de la chromatographie liquide haute performance.

### a) Dosage des protéines

Le dosage des protéines a été réalisé selon la méthode de BRADFORD (Bradford, M.M., Anal. Biochem., 1976, 72:248). L'augmentation de l'absorbance à 595 nm est proportionnelle à la concentration des protéines déterminée à l'aide d'un spectrophotomètre.

### b) Chromatographie liquide à haute performance (HPLC)

La dihydrotestostérone a été dosée par mesure spectrophotométrique après séparation par HPLC (Pompe Bischoff Model 2.200, injecteur automatique Alcoot Model 788 autosampler, colonne Ultrasep C 18 (30 cm x 0,18 cm) 6 mm de porosité, détecteur spectrophotométrique).

La détection a été réalisée à 215 nm. L'éluant utilisé a été composé d'un mélange d'hexane-isopropanol (9:1, v/v). Le flux était de 1 mL/min.

La quantification ainsi que le traitement des données ont été réalisées grâce à un logiciel informatique ICS (PiC 3).

### c) Analyse des résultats

La 5α-réductase est une enzyme qui catalyse la réaction de transformation de la testostérone (T) en DHT. Le dosage de la DHT à la fin de l'incubation nous renseigne sur l'activité de cette dernière.

### B.RESULTATS

Les résultats présentés dans la Figure 1 montrent que l'extrait de *Dianthus carthusianorum* aux concentrations 0,01% et 0,05% en poids inhibe significativement la 5α-réductase respectivement de 17% et 16% comparativement à la finastéride, produit de référence positif entraînant une inhibition de 36%.

### II. EXEMPLES

| **GEL NETTOYANT PURIFIANT :** | |
|---|---|
| | % |
| CARBOMER | 8,00 |
| LAURYL ETHER SULFATE DE SODIUM | 5,00 |
| C12-13 PARETH 3 | 0,50 |
| DECYL GLUCOSIDE | 10,00 |
| COCAMIDOPROPYL BETAINE | 5,00 |
| GLYCERINE | 5,00 |
| EXTRAIT DE *DIANTHUS CARTHUSIANORUM* | 1,00 |
| CHELATANT | 0,05 |
| SODIUM HYDROXIDE | q.s.pH |
| CONSERVATEURS | 0,05 |
| EAU DEMINERALISEE | Q.S.P. 100 |

| **LOTION POUR PEAUX GRASSES :** | |
|---|---|
| | % |
| GLYCOLS | 2,00 |
| ETHANOL | 5,00 |
| EXTRAIT DE *DIANTHUS CARTHUSIANORUM* | 1,00 |
| CHLORURE DE SODIUM | 1,00 |
| CONSERVATEURS | 0,50 |
| SOLUBILISANT | 0,30 |
| PARFUM | 0,10 |
| EAU DEMINERALISEE | Q.S.P. 100 |
| | |

| **GEL POUR PEAUX GRASSES :** | |
|---|---|
| | % |
| GLYCERINE | 3,00 |
| CARBOMER | 0,20 |
| GOMME XANTHANE | 0,17 |
| ETHANOL | 5,00 |
| EXTRAIT DE *DIANTHUS CARTHUSIANORUM* | 1,00 |
| CONSERVATEURS | 0,50 |
| SOLUBILISANT | 0,50 |
| PARFUM | 0,20 |
| EAU DEMINERALISEE | Q.S.P. 100 |
| | |

| **SERUM PEAUX GRASSES :** | |
|---|---|
| | % |
| ARISTOFLEX AVC | 1,00 |
| SEPIGEL 305 | 0,20 |
| ISONONANOATE D'ISONONYLE | 3,00 |
| SILICONE VOLATIL 0345 | 5,00 |
| DERIVE D'AMIDON MODIFIE | 2,00 |
| ACIDE SALICYLIQUE | 0,10 |
| ALCOOL 96,2° NON DENATURE | 5,00 |
| GLYCERINE | 4,00 |
| EXTRAIT DE *DIANTHUS CARTHUSIANORUM* | 2,00 |
| TROMETHAMINE | q.s.pH |
| CONSERVATEURS | q.s. |
| PARFUM | q.s. |
| EAU DEMINERALISEE | Q.S.P. 100 |
| | |

| **FLUIDE HYDRATANT MATIFIANT :** | |
|---|---|
| | % |
| GLYCERINE | 5,00 |
| PEMULEN TR1 | 0,20 |
| SEPIGEL 3 05 | 1,00 |
| ISOHEXADECANE | 2,00 |
| ISONONANOATE D'ISONONYLE | 5,00 |
| CETEARYL GLUCOSIDE | 1,00 |
| METHYL GLUCETH 20 SESQUISTEARATE | 0,50 |
| SILICONE VOLATIL 0345 | 4,00 |
| SILICONE DC 1503 | 1,00 |
| DERIVE D'AMIDON MODIFIE | 3,50 |
| EXTRAIT DE *DIANTHUS CARTHUSIANORUM* | 2,00 |
| SODIUM HYDROXIDE | 0,06 |
| CHELATANT | 0,10 |
| CONSERVATEURS | q.s. |
| EAU DEMINERALISEE | Q.S.P. 100 |
| | |

| **CREME POUR PEAUX GRASSES :** | |
|---|---|
| | % |
| CETEARYL GLUCOSIDE | 3,00 |
| MONOSTEARATE DE GLYCEROL AE | 2,00 |
| TRIGLYCERIDES C8C10 | 10,00 |
| HUILE DE SILICONE | 3,00 |
| GOMME XANTHANE | 0,10 |
| GLYCERINE | 5,00 |
| EXTRAIT DE *DIANTHUS CARTHUSIANORUM* | 2,00 |
| CONSERVATEURS | q.s. |
| PARFUM | 0,30 |
| EAU DEMINERALISEE | Q.S.P. 100 |
| | |

| **MASQUE PURIFIANT :** | |
|---|---|
| | % |
| CETEARYL GLUCOSIDE | 3,50 |
| MONOSTEARATE DE GLYCEROL AE | 2,00 |
| ALCOOL CETYLIQUE | 0,30 |
| ALCOOL STEARYLIQUE | 0,30 |
| TRIGLYCERIDES C8C10 | 9,00 |
| SILICONE VOLATIL 0345 | 4,00 |
| SEPIGEL 305 | 0,50 |
| GOMME XANTHANE | 0,15 |
| GLYCOLS | 5,00 |
| DIOXYDE DE TITANE | 2,00 |
| KAOLIN | 4,00 |
| POLYMETHYL METHACRYLATE | 0,50 |
| P.V.P. 90 | 0,50 |
| EXTRAIT DE *DIANTHUS CARTHUSIANORUM* | 1,00 |
| CONSERVATEURS | q.s. |
| CHELATANT | q.s. |
| PARFUM | q.s. |
| EAU DEMINERALISEE | Q.S.P. 100 |

## Revendications

1. Utilisation d'une composition cosmétique comprenant un extrait de *Dianthus carthusianorum* pour le soin des peaux grasses.

2. Utilisation d'une composition cosmétique comprenant un extrait de *Dianthus carthusianorum* comme agent régulateur des peaux grasses et/ou comme agent purifiant et/ou comme agent matifiant et/ou comme agent anti-brillant et/ou comme agent régulateur de la production de sébum et/ou comme agent régulateur de la prolifération des sébocytes.

3. Utilisation selon la revendication 1 ou la revendication 2, **caractérisée en ce que** la composition comprend de 0,0001% à 1% en poids d'extrait de *Dianthus carthusianorum* en poids de matière sèche par rapport au poids total de la composition.

4. Utilisation selon l'une des revendications 1 à 3, **caractérisée en ce que** la composition contient en outre au moins un actif choisi parmi un extrait de *Lamium album,* un extrait *d'hamamélis virginiana,* de la chrysine, du chlorhydrate de piridoxine et des dérivés de zinc.

5. Procédé de traitement cosmétique pour prévenir et/ou traiter les problèmes des peaux grasses, comprenant l'application sur la peau d'une composition cosmétique comprenant un extrait de *Dianthus carthusianorum* telle que définie selon l'une des revendications 1 à 4.

6. Extrait de *Dianthus carthusianorum* pour son utilisation comme agent régulateur de la production de sébum et/ou comme agent régulateur de la prolifération des sébocytes pour la prévention et/ou le traitement des peaux grasses.
